# EUROPEAN PATENT SPECIFICATION

(11) **EP 4 069 232 B1**
(45) Date of publication and mention of the grant of the patent: **24.12.2025**
(21) Application number: 20829008.0
(22) Date of filing: 02.12.2020
(51) Int. Cl.: A61K 31/496, A61K 31/445, A61K 31/13, A61K 45/06, A61P 25/28, A61P 43/00, A61K 9/00

(54) **MASUPIRDINE FOR TREATING BEHAVIORAL AND PSYCHOLOGICAL SYMPTOMS IN DEMENTIA**
MASUPIRDIN ZUR BEHANDLUNG VON VERHALTENS- UND PSYCHOLOGISCHEN SYMPTOMEN BEI DEMENZ
MASUPIRDINE POUR LE TRAITEMENT DE SYMPTÔMES COMPORTEMENTAUX ET PSYCHOLOGIQUES DE DÉMENCE

(30) Priority: 02.12.2019 IN 201941049516; 02.12.2019 IN 201941049517
(43) Date of publication of application: 12.10.2022
(73) Proprietor: Suven Life Sciences Limited, Hyderabad, Telangana 500034 (IN)
(72) Inventor: NIROGI, Ramakrishna, Hyderabad Telangana 500034 (IN); SHINDE, Anil Karbhari, Hyderabad Telangana 500034 (IN); JAYARAJAN, Pradeep, Hyderabad Telangana 500034 (IN); JETTA, Satish, Hyderabad Telangana 500034 (IN); PALACHARLA, Raghava Chowdary, Hyderabad Telangana 500034 (IN); PANDEY, Santosh Kumar, Hyderabad Telangana 500034 (IN); MOHAMMED, Abdul Rasheed, Hyderabad Telangana 500034 (IN); BENADE, Vijay Sidram, Hyderabad Telangana 500034 (IN); GOYAL, Vinod Kumar, Hyderabad Telangana 500034 (IN); SUBRAMANIAN, Ramkumar, Hyderabad Telangana 500034 (IN); RAVULA, Jyothsna, Hyderabad Telangana 500034 (IN); JASTI, Venkateswarlu, Hyderabad Telangana 500034 (IN)
(74) Representative: HGF
(86) International application number: PCT/IB2020/061383
(87) International publication number: WO 2021/111330

(56) References cited:
- WO-A1-2017/199070
- WO-A1-2017/199071
- WO-A1-2017/199072
- DEVSHI RAJAL ET AL: "Prevalence of Behavioural and Psychological Symptoms of Dementia in Individuals with Learning Disabilities", DIAGNOSTICS, vol. 5, no. 4, 1 December 2015 (2015-12-01), CH, pages 564 - 576, XP055825134, ISSN: 2075-4418, DOI: 10.3390/diagnostics5040564
- ENNACEUR ET AL: "One-trial object recognition in rats and mice: Methodological and theoretical issues", BEHAVIOURAL BRAIN RESEARCH, ELSEVIER, AMSTERDAM, NL, vol. 215, no. 2, 31 December 2010 (2010-12-31), pages 244 - 254, XP027272558, ISSN: 0166-4328, [retrieved on 20100107]
- FULLERTON TERENCE ET AL: "A Phase 2 clinical trial of PF-05212377 (SAM-760) in subjects with mild to moderate Alzheimer's disease with existing neuropsychiatric symptoms on a stable daily dose of donepezil", ALZHEIMER'S RESEARCH & THERAPY, vol. 10, no. 1, 5 August 2018 (2018-08-05), London, UK, pages 38, XP093247451, ISSN: 1758-9193, DOI: 10.1186/s13195-018-0368-9
- ANON.: "CTAD Clinical Trials on Alzheimer's Disease 2019 Conference Programme", CTAD CLINICAL TRIALS ON ALZHEIMER'S DISEASE 2019 CONFERENCE PROGRAMME, 4 December 2019 (2019-12-04), XP093291588
- ATRI A ET AL: "Memantine Added to Background Cholinesterase-Inhibitors Reduces Agitation in Alzheimer's Disease (P6.175)", NEUROLOGY, vol. 90, no. 15_supplement, 10 April 2018 (2018-04-10), XP093291543, ISSN: 0028-3878, DOI: 10.1212/WNL.90.15_supplement.P6.175
- LANG F M ET AL: "Intepirdine as adjunctive therapy to donepezil for mild-to-moderate Alzheimer's disease: A randomized, placebo-controlled, phase 3 clinical trial (MINDSET)", ALZHEIMER'S & DEMENTIA: TRANSLATIONAL RESEARCH & CLINICAL INTERVENTIONS, vol. 7, no. 1, 1 January 2021 (2021-01-01), XP093291565, ISSN: 2352-8737, DOI: 10.1002/trc2.12136
- EIKELBOOM WILLEM S. ET AL: "Neuropsychiatric and Cognitive Symptoms Across the Alzheimer Disease Clinical Spectrum : Cross-sectional and Longitudinal Associations", NEUROLOGY, vol. 97, no. 13, 28 September 2021 (2021-09-28), XP093131522, ISSN: 0028-3878, DOI: 10.1212/WNL.0000000000012598
- BANNING LEONIE C.P. ET AL: "The Association Between Biomarkers and Neuropsychiatric Symptoms Across the Alzheimer's Disease Spectrum", AMERICAN JOURNAL OF GERIATRIC PSYCHIATRY., vol. 28, no. 7, 20 February 2020 (2020-02-20), US, pages 735 - 744, XP093131514, ISSN: 1064-7481, DOI: 10.1016/j.jagp.2020.01.012
- MALPAS CHARLES B. ET AL: "The histopathological staging of tau, but not amyloid, corresponds to antemortem cognitive status, dementia stage, functional abilities and neuropsychiatric symptoms", INTERNATIONAL JOURNAL OF NEUROSCIENCE, vol. 131, no. 8, 30 April 2020 (2020-04-30), pages 800 - 809, XP093131513, ISSN: 0020-7454, DOI: 10.1080/00207454.2020.1758087
- DEVANAND DAVANGERE P. ET AL: "Associations Between Neuropsychiatric Symptoms and Neuropathological Diagnoses of Alzheimer Disease and Related Dementias", JAMA PSYCHIATRY, vol. 79, no. 4, 16 February 2022 (2022-02-16), pages 359, XP093131535, ISSN: 2168-622X, DOI: 10.1001/jamapsychiatry.2021.4363
- GARRE-OLMO JOSEP ET AL: "Grouping and Trajectories of Neuropsychiatric Symptoms in Patients with Alzheimer's Disease. Part II: Two-Year Patient Trajectories", JOURNAL OF ALZHEIMER`S DISEASE, vol. 22, no. 4, 7 January 2011 (2011-01-07), NL, pages 1169 - 1180, XP093131516, ISSN: 1387-2877, DOI: 10.3233/JAD-2010-101215
- WISE ELIZABETH A. ET AL: "Time course of neuropsychiatric symptoms and cognitive diagnosis in National Alzheimer's Coordinating Centers volunteers", ALZHEIMER'S & DEMENTIA: DIAGNOSIS, ASSESSMENT & DISEASE MONITORING, vol. 11, no. 1, 18 April 2019 (2019-04-18), pages 333 - 339, XP093131529, ISSN: 2352-8729, DOI: 10.1016/j.dadm.2019.02.006
- KRELL-ROESCH JANINA ET AL: "Cortical [beta]-amyloid burden, neuropsychiatric symptoms, and cognitive status: the Mayo Clinic Study of Aging", TRANSLATIONAL PSYCHIATRY, vol. 9, no. 1, 28 March 2019 (2019-03-28), GB, XP093131481, ISSN: 2158-3188, DOI: 10.1038/s41398-019-0456-z
- CUMMINGS JEFFREY: "Lessons Learned from Alzheimer Disease: Clinical Trials with Negative Outcomes", CTS - CLINICAL AND TRANSLATIONAL SCIENCE, vol. 11, no. 2, 1 March 2018 (2018-03-01), US, pages 147 - 152, XP093247437, ISSN: 1752-8054, DOI: 10.1111/cts.12491
- NIROGI RAMAKRISHNA ET AL: "Progress in Investigational Agents Targeting Serotonin-6 Receptors for the Treatment of Brain Disorders", BIOMOLECULES, vol. 13, no. 2, 7 February 2023 (2023-02-07), CH, pages 309, XP093247458, ISSN: 2218-273X, DOI: 10.3390/biom13020309
- ATRI A ET AL: "Effect of Idalopirdine as Adjunct to Cholinesterase Inhibitors on Change in Cognition in Patients With Alzheimer Disease Three Randomized Clinical Trials", JAMA, vol. 319, no. 2, 9 January 2011 (2011-01-09), pages 130, XP093247446, DOI: 10.1001/jama.2017.20373
- NIROGI RAMAKRISHNA ET AL: "Effect of masupirdine (SUVN-502) on cognition in patients with moderate Alzheimer's disease: A randomized, double-blind, phase 2, proof-of-concept study", ALZHEIMER'S & DEMENTIA: TRANSLATIONAL RESEARCH & CLINICAL INTERVENTIONS, vol. 8, no. 1, 30 May 2022 (2022-05-30), pages e12307, XP093133212, ISSN: 2352-8737, DOI: 10.1002/trc2.12307
- JONES ROY W: "Dimebon disappointment", ALZHEIMER'S RESEARCH & THERAPY, vol. 2, no. 5, 13 September 2010 (2010-09-13), London, UK, pages 25, XP093247455, ISSN: 1758-9193, DOI: 10.1186/alzrt49
- GRANDHI VENKATA RAMALINGAYYA ET AL: "SUVN-502: A PURE 5-HT6 RECEPTOR ANTAGONIST AMELIORATES SENILE DEMENTIA IN ANIMAL MODELS", ALZHEIMER'S & DEMENTIA: THE JOURNAL OF THE ALZHEIMER'SASSOCIATION, ELSEVIER, NEW YORK, NY, US, vol. 15, no. 7, 1 July 2019 (2019-07-01), XP085868749, ISSN: 1552-5260, [retrieved on 20191018], DOI: 10.1016/J.JALZ.2019.06.2490
- JAYARAJAN PRADEEP ET AL: "P1-302: 5-HT6 Antagonist SUVN-502 potentiates the procognitive and neurochemical effects of donepezil and memantine", ALZHEIMER'S & DEMENTIA: THE JOURNAL OF THE ALZHEIMER'SASSOCIATION, vol. 11, no. 7S_Part_10, 1 July 2015 (2015-07-01), US, pages P472 - P472, XP055783576, ISSN: 1552-5260, DOI: 10.1016/j.jalz.2015.06.516
- JAYARAJAN PRADEEP ET AL: "SUVN-502 + DONEPEZIL + MEMANTINE (TRIPLE COMBINATION): FIRST-IN-CLASS AND A PROMISING NEW APPROACH FOR THE SYMPTOMATIC TREATMENT OF ALZHEIMER'S DISEASE DEMENTIA", ALZHEIMER'S & DEMENTIA: THE JOURNAL OF THE ALZHEIMER'SASSOCIATION, ELSEVIER, NEW YORK, NY, US, vol. 15, no. 7, 1 July 2019 (2019-07-01), XP085868195, ISSN: 1552-5260, [retrieved on 20191018], DOI: 10.1016/J.JALZ.2019.06.644
- NIROGI RAMAKRISHNA ET AL: "TRIPLE THERAPY WITH MASUPIRDINE (SUVN-502), A 5-HT6 ANTAGONIST, DONEPEZIL AND MEMANTINE IN MODERATE ALZHEIMER'S DISEASE: BASELINE PATIENT CHARACTERISTICS IN A PHASE-2A STUDY", ALZHEIMER'S & DEMENTIA: THE JOURNAL OF THE ALZHEIMER'SASSOCIATION, ELSEVIER, NEW YORK, NY, US, vol. 15, no. 7, 1 July 2019 (2019-07-01), XP085870309, ISSN: 1552-5260, [retrieved on 20191018], DOI: 10.1016/J.JALZ.2019.09.026
- ATRI ALIREZA ET AL: "LBP59: Triple therapy with SUVN-502, a 5-ht6 antagonist, donepezil and memantine in moderate Alzheimer's disease: Baseline patient characteristics in phase-2a study", JOURNAL OF PREVENTION OF ALZHEIMER'S DISEASE2018SPRINGERCHE; 11TH CONFERENCE CLINICAL TRIALS ALZHEIMER'S DISEASE, EDITIONS S E R D I, FR, vol. 5, no. 1, 1 October 2018 (2018-10-01), pages S194 - S195, XP009526182, ISSN: 2426-0266, [retrieved on 20181004], DOI: 10.14283/JPAD.2018.40
- CUMMINGS J, NIROGI R, JAYARAJAN P, SHINDE A, JASTI, V: "P038: Potential benefits of masupirdine (SUVN-502) on behavioral and psychological symptoms in patients with moderate Alzheimer's disease", JOURNAL OF PREVENTION OF ALZHEIMER'S DISEASE, vol. 6, no. 1, 8 December 2019 (2019-12-08), pages S68, XP009526338, ISSN: 2426-0266, DOI: 10.14283/jpad.2019.48

## Description

### FIELD OF THE INVENTION

The present invention provides masupirdine, or a pharmaceutically acceptable salt thereof for use in a method of treating agitation/aggression, delusions, hallucinations, delusions and hallucinations, aberrant motor behavior, euphoria/elation, disinhibition, or sleep and night-time behavior change in a patient with dementia comprising administering to the patient an effective dose of masupirdine or a pharmaceutically acceptable salt thereof either alone or in combination with an acetylcholinesterase inhibitor and NMDA (N-Methyl-D-aspartate) receptor antagonist. The present invention further provides pharmaceutical compositions comprising the said compounds for use in the treatment of agitation/aggression, delusions, hallucinations, delusions and hallucinations, aberrant motor behavior, euphoria/elation, disinhibition, or sleep and night-time behavior change in a patient with dementia as described herein.

### BACKGROUND OF THE INVENTION

Dementia is a syndrome in which there is deterioration in memory, thinking, behavior and the ability to perform everyday activities. The most common form of dementia is Alzheimer's disease (AD) dementia. Other major forms of dementia include dementia associated with schizophrenia, vascular dementia (VD), Lewy body dementia (LBD), Parkinson's disease (PD) dementia, and frontotemporal dementia (FTD). Although they are pathologically different from each other, they have common symptoms which often co-exist. Behavioral and psychological symptoms of dementia (BPSD) are common symptoms observed across forms of dementia which represent heterogeneous group of non-cognitive symptoms and behaviors. Behavioral and psychological symptoms are also known as neuropsychiatric symptoms (NPS). Several studies have emphasized the role of neurochemical, neuropathological and genetic factors underlying the clinical manifestations of BPSD. It constitutes the major component of the dementia syndrome irrespective of its subtype. It is estimated that behavioral and psychological symptoms affects up to 90% of all dementia subjects over the course of their illness, and is independently associated with poor outcomes, including distress among patients, distress among caregivers, early and long-term institutionalization, misuse of medication, and increased health care costs. The behavioral and psychological symptoms are clinically as relevant as cognitive symptoms because they strongly correlate with the degree of functional and cognitive impairment. Behavioral and psychological symptoms include agitation/aggression, delusions and/or hallucinations, aberrant motor behavior, aberrant vocalizations, anxiety, euphoria/elation, irritability, depression/dysphoria, apathy, disinhibition, sleep and night-time behavior change, and appetite and eating change. Clinical evidences suggest that there is broad overlap between the behaviorally relevant circuits and networks associated with these symptoms. The Neuropsychiatric Inventory (NPI) scale is a widely used to assess NPS in patients with dementia.

Agitation/aggression, delusions and hallucinations are some of the most frequent and clinically important symptoms of BPSD. Delusions and/or hallucinations are domains of psychosis. Psychosis symptoms are common disorder across all forms of dementia with 20% to 70% prevalence (J. Cell. Mol. Med., 2012, 16, 995-1012). It is reported that, in patients with AD psychosis, an increased occurrence of severe psychosis is associated with an increased presence of delusions and hallucinations in addition to symptoms of agitation/aggression. Currently, no pharmacological treatment is approved for patients with AD psychosis specifically in patients experiencing severe psychotic symptoms.

Combination of non-pharmacological and careful use of pharmacological interventions is the recommended therapy for managing BPSD. Published literature suggests that, modest symptomatic benefit is associated with short-term treatment of patients with atypical antipsychotic agents like risperidone, olanzapine, and aripiprazole. However, benefits for longer-term treatment with these agents are less clear (Nat. Rev. Neurol., 2009, 5(5), 245-255, and N. Engl. J. Med., 2006, 355(15), 1525-1538). Moreover, the modest benefits must be balanced against significant safety concerns associated with long term use of these drugs that include risks of accelerated cognitive decline, stroke, and death. Atypical antipsychotics have been associated with statistically significant acceleration of cognitive deterioration in patients with AD. Pimavanserin, a 5-HT_{2A} antagonist was recently approved for PD psychosis shown statistically significant effect on NPI-NH psychosis scale at week 6, however, no effect was evident at week 12 indicating the absence of sustained effect (The lancet neurology, 2018, 17, 213-222). Citalopram a selective serotonin reuptake inhibitor with a 30-mg daily dose showed significant decrease in agitation in 186 patients with AD (JAMA 2014;311:682-91). Worsening of cognition and QT interval prolongation were seen in citalopram treatment group.

Given the modest efficacy of current therapies, there is an urgent medical need to identify novel pharmacological mechanism through which one can address the current limitations like durability of the effect, cognitive worsening while treating BPSD.

WO2019/008484 A1 discloses new uses of a pure 5-HT₆ receptor (5-HT₆R) antagonist in the treatment of dementia due to menopause, senile dementia, vascular dementia, chemotherapy induced cognitive impairment, and behavioral changes in dementia.

WO2017199070 discloses a triple combination of pure 5-HT₆R antagonists, acetylcholinesterase inhibitors and NMDA receptor antagonist for the treatment of cognitive disorders.

WO 2017/199071 discloses a combination of pure 5-HT₆ receptor (5-HT₆R) antagonists and acetylcholinesterase inhibitors for the treatment of cognitive disorders.

WO2017199072 discloses a combination of pure 5-HT6R antagonists and NMDA antagonist for the treatment of cognitive disorders.

The above patent publications do not disclose Masupirdine, either alone or in combination with an acetylcholinesterase inhibitor or NMDA antagonist for use in a method of treating behavioral and psychological symptoms in a patient with dementia.

Alzheimer's Dement. 2019, 15(7suppl.), P604 discloses masupirdine (SUVN-502) for use in reversing episodic and spatial memory deficits in rats in a model of senile dementia. Co-treatment of 5HT₆R antagonist, SUVN-502, with donepezil and memantine significantly potentiated the procognitive effects when compared with a combination of memantine and donepezil in object recognition task (Alzheimer's Dement. 2015, 11(7suppl.), P472*).* Co-treatment of SUVN-502 with donepezil and memantine significantly potentiated the procognitive effects as compared to memantine and donepezil treatment alone in the Morris water maze task (Alzheimer's Dement. 2019, 15(7suppl.), P269*).* SUVN-502 is in development as a novel approach in the symptomatic treatment of AD dementia as a triple therapy with SUVN-502 added to background treatment with donepezil and memantine (Alzheimer's Dement. 2019, 15(7suppl.), P1584*;* and J. Prev. Alzheimer's Dis. 2018, 5(1), S194*).* Behavioral and psychological symptoms of dementia are characterized with difference in frequency and severity of different symptoms (J. Alzheimers Dis. 2010, 22(4), 1169). Neuropsychiatric symptoms appear before a cognitive diagnosis in most people who develop cognitive decline, including both mild cognitive impairment (MCI) and dementia (Alzheimer's Dement. (Amst.) 2019, 11, 333)

The above publications do not disclose Masupirdine, either alone or in combination with donepezil or memantine, for use in a method of treating behavioral and psychological symptoms in a patient with dementia.

Masupirdine (international nonproprietary name (INN) of 1-[(2-bromophenyl)sulfonyl]-5-methoxy-3-[(4-methyl-1-piperazinyl)methyl]-1H-indole) is a pure 5-HT6R antagonist with high selectivity over receptor closely related serotonin subtypes. Masupirdine dimesylate monohydrate was evaluated in a double-blind placebo controlled, randomized, 26-week treatment phase-2 study (Clinicaltrials.gov identifier: NCT02580305) in moderate AD subjects on stable donepezil HCl and memantine HCl treatment. The primary efficacy endpoint of the trial was change from baseline at Week 26 in ADAS-Cog 11 score. The secondary outcome measures were MMSE, CDR-SB, ADCS-ADL, NPI-12, C-SDD, safety and tolerability assessment. Masupirdine was safe and well tolerated with no significant adverse events; Masupirdine did not show significant treatment advantage in cognition over placebo as measured by ADAS-Cog 11. However, significant treatment effect was observed on NPI domains as measured by the NPI-12 scale.

Subsequently, subgroup analyses were done on the phase-2 study outcome to evaluate beneficial effects on neuropsychiatric parameters. This subgroup data analyses surprisingly identified masupirdine treated patients with significant improvement in behavioral and psychological symptoms as assessed by the NPI-12 subscale scores. Masupirdine also significantly improved cognition in subjects with delusions and hallucinations (subjects with baseline symptoms) as assessed by the ADAS-Cog 11 scale.

### SUMMARY OF THE INVENTION

The invention is defined by the claims. Any subject-matter falling outside the scope of the claims is provided for information purposes only. Any references in the description to methods of treatment refer to the compounds, pharmaceutical compositions and medicaments of the present invention for use in a method for treatment of the human or animal body by therapy.

The present invention provides masupirdine or a pharmaceutically acceptable salt thereof, for use in a method of treating agitation/aggression, delusions, hallucinations, delusions and hallucinations, aberrant motor behavior, euphoria/elation, disinhibition, or sleep and night-time behavior change in a patient with dementia, comprising administering to the patient an effective dose of masupirdine or a pharmaceutically acceptable salt thereof, wherein the effective dose of masupirdine or a pharmaceutically acceptable salt thereof is selected from 25 mg to 125 mg, wherein the dementia is selected from Alzheimer's disease dementia, dementia associated with schizophrenia, Parkinson's disease dementia, Lewy body dementia, vascular dementia, or frontotemporal dementia.

In an aspect, the present invention provides a pharmaceutical composition, comprising an effective dose of masupirdine or a pharmaceutically acceptable salt thereof, for use in the treatment of agitation/aggression, delusions, hallucinations, delusions and hallucinations, aberrant motor behavior, euphoria/elation, disinhibition, or sleep and night-time behavior change, in a patient with dementia, wherein the effective dose of masupirdine or a pharmaceutically acceptable salt thereof is selected from 25 mg to 125 mg, wherein the dementia is selected from Alzheimer's disease dementia, dementia associated with schizophrenia, Parkinson's disease dementia, Lewy body dementia, vascular dementia, or frontotemporal dementia.

In an embodiment, the present invention relates to a pharmaceutical composition comprising an effective dose of masupirdine or a pharmaceutically acceptable salt thereof and one or more pharmaceutically acceptable excipients for use in the treatment of agitation/aggression, delusions, hallucinations, delusions and hallucinations, aberrant motor behavior, euphoria/elation, disinhibition, or sleep and night-time behavior change in a patient with dementia, wherein the effective dose of masupirdine or a pharmaceutically acceptable salt thereof is selected from 25 mg to 125 mg, wherein the dementia is selected from Alzheimer's disease dementia, dementia associated with schizophrenia, Parkinson's disease dementia, Lewy body dementia, vascular dementia, or frontotemporal dementia.

The acetylcholinesterase inhibitor is selected from donepezil, rivastigmine, galantamine or pharmaceutically acceptable salts thereof.

The NMDA receptor antagonist is memantine or a pharmaceutically acceptable salt thereof.

### BRIEF DESCRIPTION OF THE DIAGRAMS

Figure 1: Depicts effect of masupirdine on the total NPI scores.
Figure 2: Depicts effect of masupirdine in patient with agitation/aggression.
Figure 3: Depicts effect of masupirdine in patients with agitation/aggression.
Figure 4: Depicts effect of masupirdine in patients with agitation/aggression.
Figure 5: Depicts effect of masupirdine in patients with agitation/aggression, and/or aberrant motor behavior, and/or sleep and nighttime behavior change.
Figure 6: Depicts effect of masupirdine in patients with delusions and/or hallucinations.
Figure 7: Depicts effect of masupirdine on ADAScog-11 scores in patients with delusions and/or hallucinations.
Figure 8: Depicts effect of masupirdine in patients with disinhibition.
Figure 9: Depicts effect of masupirdine in patients with euphoria/elation.
Figure 10: Depicts effect of masupirdine in patients with aberrant motor behavior.
Figure 11: Depicts effect of masupirdine in patients with sleep and nighttime behavior.

### DETAILED DESCRIPTION

Unless otherwise stated, the following terms used in the specification and claims have the meanings given below:
The term, "5-HT₆ receptor antagonist" as used herein refers to a ligand or small molecule that shows affinity towards 5-HT₆ receptor, blocks or inhibits the function/binding of 5-HT₆ receptor agonist.

The term, "pure 5-HT₆ receptor antagonist" as used herein refers to a 5-HT₆ receptor antagonist that has very high selectivity (>250 fold) over closely related serotonin subtypes like 5-HT_{1A}, 5-HT_{1B}, 5-HT_{1D}, 5-HT_{2A}, 5-HT_{2C}, 5-HT₄, 5-HT_{5A} and 5-HT₇.

Example of pure 5-HT₆ receptor antagonist is 1-[(2-Bromophenyl)sulfonyl]-5-methoxy-3-[(4-methyl-1-piperazinyl)methyl]-1H-indole.

The international nonproprietary name (INN) for 1-[(2-Bromophenyl)sulfonyl]-5-methoxy-3-[(4-methyl-1-piperazinyl)methyl]-1H-indole is Masupirdine.

Example of pharmaceutically acceptable salt of pure 5-HT₆ receptor antagonist, masupirdine is dimesylate monohydrate.

Masupirdine dimesylate monohydrate is also known as SUVN-502 which has the chemical structure as shown below.

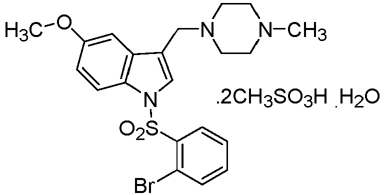

Masupirdine is commonly administered as masupirdine dimesylate monohydrate. Compound, masupirdine dimesylate monohydrate and its preparation have been described in patents US7875605 and US9540321 and in article J. Med. Chem. 2017, 60, 5, 1843-1859*.*

The term, "active compound(s)" or "compound(s)" as used herein refers to a 5-HT₆ receptor antagonist or an acetylcholinesterase inhibitor or NMDA receptor antagonist. Preferably the 5-HT₆ receptor antagonist is (1-[(2-Bromophenyl)sulfonyl]-5-methoxy-3-[(4-methyl-1-piperazinyl)methyl]-1H-indole) or a pharmaceutically acceptable salt, the acetylcholinesterase inhibitor is donepezil, rivastigmine, galantamine or pharmaceutically acceptable salts thereof, and NMDA receptor antagonist is memantine or pharmaceutically acceptable salts thereof.

The term, "acetylcholinesterase inhibitor" as used herein is a chemical or drug that inhibits acetylcholinesterase enzyme from breaking down acetylcholine, thereby increases levels and duration of action of the neurotransmitter acetylcholine. Examples of acetyl cholinesterase inhibitors are; donepezil, rivastigmine, and galantamine. Preferably, the acetylcholinesterase inhibitor is donepezil, rivastigmine, galantamine, or pharmaceutically acceptable salts thereof.

Donepezil is a drug approved for the treatment of mild, moderate, and severe dementia of AD. Donepezil is a reversible inhibitor of the enzyme acetylcholinesterase and sold under trade name Aricept^{®} as hydrochloride salt.

Rivastigmine is a drug approved for treatment of mild, moderate and severe dementia of AD. Rivastigmine is a reversible cholinesterase inhibitor and sold under trade name Exelon^{®} and Exelon Patch^{®} as tartrate salt.

Galantamine is a drug approved for treatment of mild, moderate and severe dementia of AD. Galantamine, a reversible, competitive acetylcholinesterase inhibitor and sold under trade name Razadyne^{®} as hydrobromide salt.

The term, "NMDA receptor antagonist" as used herein refers to class of compounds which act on glutamatergic system by inhibiting the NMDA receptor. Example of NMDA receptor antagonist is memantine. Memantine is a drug approved for treatment of moderate to severe dementia of the AD. Memantine is sold under trade name Namenda^{®} and Namenda XR^{®} as hydrochloride salt.

The term, "modified Intent-To-Treat (mITT)" is defined as all randomized subjects who receive at least one dose of study medication and have one postbaseline evaluation of the primary efficacy variable.

The term "placebo" as used herein is a tablet with an inert substance and has no therapeutic value which is identically appearing to masupirdine dimesylate monohydrate tablets in color, smell, taste, and appearance and has been taken along with donepezil and memantine.

The term "baseline domain score" refers to the score of total NPI-12 domains or the score of individual domain measured prior to the administration of masupirdine at baseline.

The phrase, "an effective amount" or "an effective dose" is defined as an amount of a compound of the present invention that (i) treats the particular disease, condition, or disorder, (ii) eliminates one or more symptoms of the particular disease, condition or disorder and (iii) delays the onset of one or more symptoms of the particular disease, condition or disorder described herein.

The term, "pharmaceutically acceptable salt(s)" as used herein refers to salts of the active compound and are prepared by reacting masupirdine, donepezil or memantine with appropriate organic or inorganic acids or acid derivatives. The pharmaceutically acceptable salts include but are not limited to, dimesylate, dihydrochloride, hydrochloride, oxalate, succinate, and tartrate and alike. Preferably, the pharmaceutically acceptable salts are dihydrochlorides and dimesylates. More preferably, the pharmaceutically acceptable salt is dimesylate or hydrochloride.

The term, "patient(s)" or "subject(s)" as used herein refers to an animal. Preferably the term "patient(s)" or "subject(s)" refers to a mammal. More preferably the term "patient(s)" or "subject(s)" refers to humans.

The term "behavioral and psychological symptoms", also known as neuropsychiatric symptoms (NPS), refers to heterogeneous group of non-cognitive symptoms and behaviors occurring in subjects with dementia. BPSD constitute a major component of the dementia syndrome irrespective of its subtype. Behavioral and psychological symptoms include agitation/aggression, delusions and/or hallucinations, aberrant motor behavior, aberrant vocalizations, anxiety, euphoria/elation, irritability, depression/dysphoria, apathy, disinhibition, sleep and night-time behavior change, and appetite and eating change. It also refers to any physical or verbal behavior of dementia patients which may result in hurting or repelling others, and includes aggressive behaviors such as beating, kicking, biting, and screaming.

The term "dementia" includes Alzheimer's disease (AD) dementia, dementia associated with schizophrenia, Parkinson's disease (PD) dementia, Lewy body dementia (LBD), vascular dementia (VD), and frontotemporal dementia (FTD).

The term, "Alzheimer's disease (AD)" as used herein refers to a dementia that causes problems with memory, thinking, and behavior. The Alzheimer's disease can be mild, moderate, or severe.

The term "agitation/aggression" as used herein refers to "agitation and/or aggression".

The term "delusion and/or hallucinations" or "psychosis" as used herein refers to "delusions or hallucinations", or "delusions and hallucinations".

The term, "treatment' or 'treating" as used herein refers to any treatment of a disease in a mammal, including: (a) slowing or arresting the development of clinical symptoms; and/or (b) causing the regression of clinical symptoms.

### Embodiments

The present invention is defined by the claims.The present invention relates to masupirdine or a pharmaceutically acceptable salt thereof, for use in a method of treating agitation/aggression, delusions, hallucinations, delusions and hallucinations, aberrant motor behavior, euphoria/elation, disinhibition, or sleep and night-time behavior change in patients with dementia, comprising administering to the patient an effective dose of masupirdine or a pharmaceutically acceptable salt thereof, wherein the effective dose of masupirdine or a pharmaceutically acceptable salt thereof is selected from 25 mg to 125 mg, wherein the dementia is selected from Alzheimer's disease dementia, dementia associated with schizophrenia, Parkinson's disease dementia, Lewy body dementia, vascular dementia, or frontotemporal dementia.

The present invention also relates to a pharmaceutical composition comprising an effective dose of masupirdine or a pharmaceutically acceptable salt thereof for use in the treatment of agitation/aggression, delusions, hallucinations, delusions and hallucinations, aberrant motor behavior, euphoria/elation, disinhibition, or sleep and night-time behavior change, in patients with dementia, wherein the effective dose of masupirdine or a pharmaceutically acceptable salt thereof is selected from 25 mg to 125 mg, wherein the dementia is selected from Alzheimer's disease dementia, dementia associated with schizophrenia, Parkinson's disease dementia, Lewy body dementia, vascular dementia, or frontotemporal dementia.

In another embodiment, the present invention provides masupirdine or a pharmaceutically acceptable salt thereof, for use in a method of treating agitation/aggression, delusions, hallucinations, delusions and hallucinations, aberrant motor behavior, euphoria/elation, disinhibition, or sleep and night-time behavior change, in patients with dementia, comprising administering an effective dose of masupirdine or a pharmaceutically acceptable salt thereof in combination with donepezil or a pharmaceutically acceptable salt thereof and memantine or a pharmaceutically acceptable salt thereof, wherein the effective dose of masupirdine or a pharmaceutically acceptable salt thereof is selected from 25 mg to 125 mg, wherein the dementia is selected from Alzheimer's disease dementia, dementia associated with schizophrenia, Parkinson's disease dementia, Lewy body dementia, vascular dementia, or frontotemporal dementia.

In another embodiment, the present invention relates to a pharmaceutical composition comprising an effective dose of masupirdine or a pharmaceutically acceptable salt thereof, donepezil or a pharmaceutically acceptable salt thereof and memantine or a pharmaceutically acceptable salt thereof for use in the treatment of agitation/aggression, delusions, hallucinations, delusions and hallucinations, aberrant motor behavior, euphoria/elation, disinhibition, or sleep and night-time behavior change in patients with dementia, wherein the effective dose of masupirdine or a pharmaceutically acceptable salt thereof is selected from 25 mg to 125 mg, wherein the dementia is selected from Alzheimer's disease dementia, dementia associated with schizophrenia, Parkinson's disease dementia, Lewy body dementia, vascular dementia, or frontotemporal dementia.

In another embodiment, the present invention provides masupirdine or a pharmaceutically acceptable salt thereof for use in a method of treating agitation/aggression, delusions, hallucinations, delusions and hallucinations, aberrant motor behavior, euphoria/elation, disinhibition, or sleep and night-time behavior change in patients with Alzheimer's disease dementia, comprising administering to the patients an effective dose of masupirdine or a pharmaceutically acceptable salt thereof, wherein the effective dose of masupirdine or a pharmaceutically acceptable salt thereof is selected from 25 mg to 125 mg.

In another embodiment, the present invention provides masupirdine or a pharmaceutically acceptable salt thereof for use in a method of treating agitation/aggression, delusions, hallucinations, delusions and hallucinations, aberrant motor behavior, euphoria/elation, disinhibition, or sleep and night-time behavior change in patients with dementia associated with schizophrenia, comprising administering to the patients an effective dose of masupirdine or a pharmaceutically acceptable salt thereof, wherein the effective dose of masupirdine or a pharmaceutically acceptable salt thereof is selected from 25 mg to 125 mg.

In another embodiment, the present invention provides masupirdine or a pharmaceutically acceptable salt thereof for use in a method of treating agitation/aggression, delusions, hallucinations, delusions and hallucinations, aberrant motor behavior, euphoria/elation, disinhibition, or sleep and night-time behavior change in patients with Parkinson's disease dementia, comprising administering to the patients an effective dose of masupirdine or a pharmaceutically acceptable salt thereof, wherein the effective dose of masupirdine or a pharmaceutically acceptable salt thereof is selected from 25 mg to 125 mg.

In another embodiment, the present invention provides masupirdine or a pharmaceutically acceptable salt thereof for use in a method of treating agitation/aggression, delusions, hallucinations, delusions and hallucinations, aberrant motor behavior, euphoria/elation, disinhibition, or sleep and night-time behavior change in patients with Lewy body dementia, comprising administering to the patients an effective dose of masupirdine or a pharmaceutically acceptable salt thereof, wherein the effective dose of masupirdine or a pharmaceutically acceptable salt thereof is selected from 25 mg to 125 mg.

In another embodiment, the present invention provides masupirdine or a pharmaceutically acceptable salt thereof for use in a method of treating agitation/aggression, delusions, hallucinations, delusions and hallucinations, aberrant motor behavior, euphoria/elation, disinhibition, or sleep and night-time behavior change in patients with frontotemporal dementia, comprising administering to the patients an effective dose of masupirdine or a pharmaceutically acceptable salt thereof, wherein the effective dose of masupirdine or a pharmaceutically acceptable salt thereof is selected from 25 mg to 125 mg.

In another embodiment, the present invention provides masupirdine or a pharmaceutically acceptable salt thereof for use in a method of treating agitation/aggression, delusions, hallucinations, delusions and hallucinations, aberrant motor behavior, euphoria/elation, disinhibition, or sleep and night-time behavior change in patients with Alzheimer's disease dementia, comprising administering an effective dose of masupirdine or a pharmaceutically acceptable salt thereof in combination with donepezil or a pharmaceutically acceptable salt thereof and memantine or a pharmaceutically acceptable salt thereof, wherein the effective dose of masupirdine or a pharmaceutically acceptable salt thereof is selected from 25 mg to 125 mg.

In another embodiment, the present invention provides masupirdine or a pharmaceutically acceptable salt thereof for use in a method of treating agitation/aggression, delusions, hallucinations, delusions and hallucinations, aberrant motor behavior, euphoria/elation, disinhibition, or sleep and night-time behavior change in patients with dementia associated with schizophrenia, comprising administering an effective dose of masupirdine or a pharmaceutically acceptable salt thereof in combination with donepezil or a pharmaceutically acceptable salt thereof and memantine or a pharmaceutically acceptable salt thereof, wherein the effective dose of masupirdine or a pharmaceutically acceptable salt thereof is selected from 25 mg to 125 mg.

In another embodiment, the present invention provides masupirdine or a pharmaceutically acceptable salt thereof for use in a method of treating agitation/aggression, delusions, hallucinations, delusions and hallucinations, aberrant motor behavior, euphoria/elation, disinhibition, or sleep and night-time behavior change in patients with Parkinson's disease dementia, comprising administering an effective dose of masupirdine or a pharmaceutically acceptable salt thereof in combination with donepezil or a pharmaceutically acceptable salt thereof and memantine or a pharmaceutically acceptable salt thereof, wherein the effective dose of masupirdine or a pharmaceutically acceptable salt thereof is selected from 25 mg to 125 mg.

In another embodiment, the present invention provides masupirdine or a pharmaceutically acceptable salt thereof for use in a method of treating agitation/aggression, delusions, hallucinations, delusions and hallucinations, aberrant motor behavior, euphoria/elation, disinhibition, or sleep and night-time behavior change in patients with Lewy body dementia, comprising administering an effective dose of masupirdine or a pharmaceutically acceptable salt thereof in combination with donepezil or a pharmaceutically acceptable salt thereof and memantine or a pharmaceutically acceptable salt thereof, wherein the effective dose of masupirdine or a pharmaceutically acceptable salt thereof is selected from 25 mg to 125 mg.

In another embodiment, the present invention provides masupirdine or a pharmaceutically acceptable salt thereof for use in a method of treating agitation/aggression, delusions, hallucinations, delusions and hallucinations, aberrant motor behavior, euphoria/elation, disinhibition, or sleep and night-time behavior change in patients with vascular dementia, comprising administering an effective dose of masupirdine or a pharmaceutically acceptable salt thereof in combination with donepezil or a pharmaceutically acceptable salt thereof and memantine or a pharmaceutically acceptable salt thereof, wherein the effective dose of masupirdine or a pharmaceutically acceptable salt thereof is selected from 25 mg to 125 mg.

In another embodiment, the present invention provides masupirdine or a pharmaceutically acceptable salt thereof for use in a method of treating agitation/aggression, delusions, hallucinations, delusions and hallucinations, aberrant motor behavior, euphoria/elation, disinhibition, or sleep and night-time behavior change in patients with frontotemporal dementia, comprising administering an effective dose of masupirdine or a pharmaceutically acceptable salt thereof in combination with donepezil or a pharmaceutically acceptable salt thereof and memantine or a pharmaceutically acceptable salt thereof, wherein the effective dose of masupirdine or a pharmaceutically acceptable salt thereof is selected from 25 mg to 125 mg.

In certain embodiments, the behavioral and psychological symptom in patient with dementia is agitation/aggression.

In certain embodiments, the behavioral and psychological symptom in patient with dementia is delusions.

In certain embodiments, the behavioral and psychological symptom in patient with dementia is hallucinations.

In certain embodiments, the behavioral and psychological symptom in patient with dementia is delusions and/or hallucinations.

In certain embodiments, the behavioral and psychological symptom in patient with dementia is aberrant motor behavior.

In certain embodiments, the behavioral and psychological symptom in patient with dementia is euphoria/elation.

In certain embodiments, the behavioral and psychological symptom in patient with dementia is disinhibition.

In certain embodiments, the behavioral and psychological symptom in patient with dementia is sleep and night-time behavior change.

In certain embodiments, the behavioral and psychological symptoms in patient with dementia are agitation/aggression, aberrant motor behavior, and sleep and night-time behavior change.

In certain embodiments, the behavioral and psychological symptom in patient with Alzheimer's disease dementia is agitation/aggression.

In certain embodiments, the behavioral and psychological symptom in patient with Alzheimer's disease dementia is delusions.

In certain embodiments, the behavioral and psychological symptom in patient with Alzheimer's disease dementia is hallucinations.

In certain embodiments, the behavioral and psychological symptom in patient with Alzheimer's disease dementia is delusions and/or hallucinations.

In certain embodiments, the behavioral and psychological symptom in patient with Alzheimer's disease dementia is aberrant motor behavior.

In certain embodiments, the behavioral and psychological symptom in patient with Alzheimer's disease dementia is euphoria/elation.

In certain embodiments, the behavioral and psychological symptom in patient with Alzheimer's disease dementia is disinhibition.

In certain embodiments, the behavioral and psychological symptom in patient with Alzheimer's disease dementia is sleep and night-time behavior change.

In certain embodiments, the behavioral and psychological symptoms in patient with Alzheimer's disease dementia are agitation/aggression, aberrant motor behavior, and sleep and nigh-time behavior change.

In certain embodiments, the pharmaceutically acceptable salt of donepezil is hydrochloride.

In certain embodiments, the pharmaceutically acceptable salt of memantine is hydrochloride.

In another embodiment, the present invention relates to masupirdine, or a pharmaceutically acceptable salt thereof, for use in a method of treating agitation/aggression in a patient with Alzheimer's disease dementia, comprising administering an effective dose of masupirdine or a pharmaceutically acceptable salt thereof.

In another embodiment, the present invention relates to masupirdine, or a pharmaceutically acceptable salt thereof, for use in a method of treating delusions in a patient with Alzheimer's disease dementia, comprising administering an effective dose of masupirdine or a pharmaceutically acceptable salt thereof.

In another embodiment, the present invention relates to masupirdine, or a pharmaceutically acceptable salt thereof, for use in a method of treating hallucinations in a patient with Alzheimer's disease dementia, comprising administering an effective dose of masupirdine or a pharmaceutically acceptable salt thereof.

In another embodiment, the present invention relates to masupirdine, or a pharmaceutically acceptable salt thereof, for use in a method of treating delusions and/or hallucinations in a patient with Alzheimer's disease dementia, comprising administering an effective dose of masupirdine or a pharmaceutically acceptable salt thereof.

In another embodiment, the present invention relates to masupirdine, or a pharmaceutically acceptable salt thereof, for use in a method of treating aberrant motor behavior change in a patient with Alzheimer's disease dementia, comprising administering an effective dose of masupirdine or a pharmaceutically acceptable salt thereof.

In another embodiment, the present invention relates to masupirdine, or a pharmaceutically acceptable salt thereof, for use in a method of treating euphoria/elation in a patient with Alzheimer's disease dementia, comprising administering an effective dose of masupirdine or a pharmaceutically acceptable salt thereof.

In another embodiment, the present invention relates to masupirdine, or a pharmaceutically acceptable salt thereof, for use in a method of treating disinhibition in a patient with Alzheimer's disease dementia, comprising administering an effective dose of masupirdine or a pharmaceutically acceptable salt thereof.

In another embodiment, the present invention relates to masupirdine, or a pharmaceutically acceptable salt thereof, for use in a method of treating sleep and night-time behavior change in a patient with Alzheimer's disease dementia, comprising administering an effective dose of masupirdine or a pharmaceutically acceptable salt thereof.

The treatment comprises administering to the patient 25 mg to 125 mg of masupirdine or a pharmaceutically acceptable salt thereof.

In another embodiment, the treatment comprises administering to the patient 50 mg to 100 mg of masupirdine or a pharmaceutically acceptable salt thereof.

In another embodiment, the treatment comprises administering to the patient 25 mg to 75 mg of masupirdine or a pharmaceutically acceptable salt thereof.

In another embodiment, the treatment comprises administering to the patient 75 mg to 125 mg of masupirdine or a pharmaceutically acceptable salt thereof.

In another embodiment, the treatment comprising administering to the patients 25 mg to 50 mg of masupirdine or pharmaceutically acceptable salts thereof.

In certain embodiments, the patient has a baseline score of 1 or greater in the agitation/aggression subscale of the NPI-12.

In certain embodiments, the patient has a baseline score of 1 or greater in the delusions and/or hallucinations subscale of the NPI-12.

In certain embodiments, the patient has a baseline score of 1 or greater in the agitation/aggression, and/or aberrant motor behavior, and/or sleep and nighttime behavior change subscale of the NPI-12.

In certain embodiments, the patient has a baseline score of 1 or greater in the sleep and night-time behavior change subscale of the NPI-12.

In another embodiment, the treatment comprises administering to the patient 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, or 125 mg of masupirdine or a pharmaceutically acceptable salt thereof.

In another embodiment, the treatment comprises administering to the patient 25 mg, 50 mg, 75 mg, 100 mg, or 125 mg of masupirdine or a pharmaceutically acceptable salt thereof.

In some embodiments, the pharmaceutically acceptable salt of masupirdine is selected from mesylate, hydrochloride, oxalate, succinate, and tartrate salts.

In certain embodiments, the pharmaceutically acceptable salt of masupirdine is dimesylate monohydrate.

In another embodiment, the treatment comprises administering to the patient 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, or 125 mg of masupirdine dimesylate monohydrate.

In another embodiment, the treatment comprises administering to the patient 25 mg, 50 mg, 75 mg, 100 mg, or 125 mg of masupirdine dimesylate monohydrate.

In certain embodiments, the treatment comprises administering to the patient 50 mg of masupirdine dimesylate monohydrate.

In certain embodiments, the treatment comprises administering to the patient 100 mg of masupirdine dimesylate monohydrate.

In another embodiment, the masupirdine or a pharmaceutically acceptable salt thereof can be co-administered with the donepezil or a pharmaceutically acceptable salt thereof and memantine or a pharmaceutically acceptable salt thereof, to the patient at daily dose of 25 mg to 125 mg.

In another embodiment, the masupirdine or a pharmaceutically acceptable salt thereof can be co-administered with the donepezil or a pharmaceutically acceptable salt thereof and memantine or a pharmaceutically acceptable salt thereof, to the patient at daily dose of 50 mg to 100 mg.

In another embodiment, the masupirdine or a pharmaceutically acceptable salt thereof can be co-administered with the donepezil or a pharmaceutically acceptable salt thereof and memantine or a pharmaceutically acceptable salt thereof, to the patient at daily dose of 25 mg to 75 mg.

In another embodiment, the masupirdine or a pharmaceutically acceptable salt thereof can be co-administered with the donepezil or a pharmaceutically acceptable salt thereof and memantine or a pharmaceutically acceptable salt thereof, to the patient at daily dose of 75 mg to 125 mg.

In another embodiment, the masupirdine, or pharmaceutically acceptable salts thereof can be co-administered with the donepezil or a pharmaceutically acceptable salt thereof and memantine or a pharmaceutically acceptable salt thereof, to the patient at daily dose of 25 mg to 50 mg.

In another embodiment, the masupirdine, or a pharmaceutically acceptable salt thereof can be co-administered with the donepezil or a pharmaceutically acceptable salt thereof and memantine or a pharmaceutically acceptable salt thereof, to the patient at daily dose of 100 mg.

In another embodiment, the masupirdine or a pharmaceutically acceptable salt thereof can be co-administered with the donepezil or a pharmaceutically acceptable salt thereof and memantine or a pharmaceutically acceptable salt thereof, to the patient at daily dose of 75 mg.

In another embodiment, the masupirdine or a pharmaceutically acceptable salt thereof can be co-administered with the donepezil or a pharmaceutically acceptable salt thereof and memantine or a pharmaceutically acceptable salt thereof, to the patient at daily dose of 50 mg.

In another embodiment, the masupirdine or a pharmaceutically acceptable salt thereof can be co-administered with the donepezil or a pharmaceutically acceptable salt thereof and memantine or a pharmaceutically acceptable salt thereof, to the patient at daily dose of 25 mg.

In another embodiment, the donepezil or a pharmaceutically acceptable salt thereof can be co-administered with the masupirdine or a pharmaceutically acceptable salt thereof and memantine or a pharmaceutically acceptable salt thereof, to the patient at daily dose of 5 mg to 23 mg.

In another embodiment, the donepezil or a pharmaceutically acceptable salt thereof can be co-administered with masupirdine or a pharmaceutically acceptable salt thereof and memantine or a pharmaceutically acceptable salt thereof, to the patient at daily dose of 10 mg to 23 mg.

In another embodiment, donepezil or a pharmaceutically acceptable salt thereof can be co-administered with the masupirdine or a pharmaceutically acceptable salt thereof and memantine or a pharmaceutically acceptable salt thereof, to the patient at daily dose of 10 mg.

In another embodiment, the memantine or a pharmaceutically acceptable salt thereof can be co-administered with the masupirdine or a pharmaceutically acceptable salt thereof and donepezil or a pharmaceutically acceptable salt thereof, to the patients at daily dose of 5 mg to 28 mg.

In another embodiment, the memantine or a pharmaceutically acceptable salt thereof can be co-administered with the masupirdine or a pharmaceutically acceptable salt thereof and donepezil or a pharmaceutically acceptable salt thereof, to the patients at daily dose of 10 mg to 28 mg.

In another embodiment, the memantine or a pharmaceutically acceptable salt thereof can be co-administered with the masupirdine or a pharmaceutically acceptable salt thereof and donepezil or a pharmaceutically acceptable salt thereof, to the patients at daily dose of 14 mg to 28 mg.

In another embodiment, the memantine or a pharmaceutically acceptable salt thereof can be co-administered with the masupirdine or a pharmaceutically acceptable salt thereof and donepezil or a pharmaceutically acceptable salt thereof, to the patients at daily dose of 21 mg to 28 mg.

In another embodiment, the memantine or a pharmaceutically acceptable salt thereof can be co-administered with the masupirdine or a pharmaceutically acceptable salt thereof and donepezil or a pharmaceutically acceptable salt thereof, to the patients at daily dose of 20 mg.

In another embodiment, the memantine or a pharmaceutically acceptable salt thereof can be co-administered with the masupirdine or a pharmaceutically acceptable salt thereof and donepezil or a pharmaceutically acceptable salt thereof, to the patients at daily dose of 21 mg.

In another embodiment, the memantine or a pharmaceutically acceptable salt thereof can be co-administered with the masupirdine or a pharmaceutically acceptable salt thereof and donepezil or a pharmaceutically acceptable salt thereof, to the patients at daily dose of 28 mg.

In another embodiment, the memantine or a pharmaceutically acceptable salt thereof can be co-administered with the masupirdine or a pharmaceutically acceptable salt thereof and donepezil or a pharmaceutically acceptable salt thereof, to the patients at daily dose of 10 mg twice daily dosing.

In another embodiment, the masupirdine or a pharmaceutically acceptable salt thereof can be administered to the patient on stable treatment with donepezil or a pharmaceutically acceptable salt thereof and memantine or a pharmaceutically acceptable salt thereof.

In another embodiment, the masupirdine or a pharmaceutically acceptable salt thereof, donepezil or a pharmaceutically acceptable salt thereof and memantine or a pharmaceutically acceptable salt thereof can be administered to the patient simultaneously, separately, or sequentially.

In another embodiment, the present invention relates to masupirdine or a pharmaceutically acceptable salt thereof for use in a method of treating agitation/aggression in patients with Alzheimer's disease dementia, comprising administering 100 mg of masupirdine dimesylate monohydrate.

In another embodiment, the present invention relates to masupirdine or a pharmaceutically acceptable salt thereof for use in a method of treating agitation/aggression in patients with Alzheimer's disease dementia, comprising administering 50 mg of masupirdine dimesylate monohydrate.

In another embodiment, the present invention relates to masupirdine or a pharmaceutically acceptable salt thereof for use in a method of treating delusions in patients with Alzheimer's disease dementia, comprising administering 100 mg of masupirdine dimesylate monohydrate.

In another embodiment, the present invention relates to masupirdine or a pharmaceutically acceptable salt thereof for use in a method of treating hallucinations in patients with Alzheimer's disease dementia, comprising administering 100 mg of masupirdine dimesylate monohydrate.

In another embodiment, the present invention relates to masupirdine or a pharmaceutically acceptable salt thereof for use in a method of treating delusions and/or hallucinations in patients with Alzheimer's disease dementia, comprising administering 100 mg of masupirdine dimesylate monohydrate.

In another embodiment, the present invention relates to masupirdine or a pharmaceutically acceptable salt thereof for use in a method of treating delusions in patients with Alzheimer's disease dementia, comprising administering 50 mg of masupirdine dimesylate monohydrate.

In another embodiment, the present invention relates to masupirdine or a pharmaceutically acceptable salt thereof for use in a method of treating hallucinations in patients with Alzheimer's disease dementia, comprising administering 50 mg of masupirdine dimesylate monohydrate.

In another embodiment, the present invention relates to masupirdine or a pharmaceutically acceptable salt thereof for use in a method of treating delusions and/or hallucinations in patients with Alzheimer's disease dementia, comprising administering 50 mg of masupirdine dimesylate monohydrate.

In another embodiment, the present invention relates to masupirdine or a pharmaceutically acceptable salt thereof for use in a method of treating aberrant motor behavior in patients with Alzheimer's disease dementia, comprising administering 100 mg of masupirdine dimesylate monohydrate.

In another embodiment, the present invention relates to masupirdine or a pharmaceutically acceptable salt thereof for use in a method of treating aberrant motor behavior in patients with Alzheimer's disease dementia, comprising administering 50 mg of masupirdine dimesylate monohydrate.

In another embodiment, the present invention relates to masupirdine or a pharmaceutically acceptable salt thereof for use in a method of treating euphoria/elation in patients with Alzheimer's disease dementia, comprising administering 100 mg of masupirdine dimesylate monohydrate.

In another embodiment, the present invention relates to masupirdine or a pharmaceutically acceptable salt thereof for use in a method of treating euphoria/elation in patients with Alzheimer's disease dementia, comprising administering 50 mg of masupirdine dimesylate monohydrate.

In another embodiment, the present invention relates to masupirdine or a pharmaceutically acceptable salt thereof for use in a method of treating disinhibition in patients with Alzheimer's disease dementia, comprising administering 100 mg of masupirdine dimesylate monohydrate.

In another embodiment, the present invention relates to masupirdine or a pharmaceutically acceptable salt thereof for use in a method of treating disinhibition in patients with Alzheimer's disease dementia, comprising administering 50 mg of masupirdine dimesylate monohydrate.

In another embodiment, the present invention relates to masupirdine or a pharmaceutically acceptable salt thereof for use in a method of treating sleep and night-time behavior change in patients with Alzheimer's disease dementia, comprising administering 100 mg of masupirdine dimesylate monohydrate.

In another embodiment, the present invention relates to masupirdine or a pharmaceutically acceptable salt thereof for use in a method of treating sleep and night-time behavior change in patients with Alzheimer's disease dementia, comprising administering 50 mg of masupirdine dimesylate monohydrate.

In embodiments, the masupirdine or a pharmaceutically acceptable salt thereof of the present invention is normally administered to the patient by formulating into a pharmaceutical composition in accordance with standard pharmaceutical practice.

In embodiments, the masupirdine or a pharmaceutically acceptable salt thereof in combination with donepezil or a pharmaceutically acceptable salt thereof and memantine or a pharmaceutically acceptable salt thereof of the present invention normally administered to the patient by formulating into a pharmaceutical composition in accordance with standard pharmaceutical practice.

The pharmaceutical compositions of the present invention may be formulated in a conventional manner using one or more pharmaceutically acceptable excipients. The pharmaceutically acceptable excipients are diluents, disintegrants, binders, lubricants, glidants, polymers, coating agents, solvents, co-solvents, preservatives, wetting agents, thickening agents, antifoaming agents, sweetening agents, flavouring agents, antioxidants, colorants, solubilizers, plasticizer, dispersing agents and the like. Excipients are selected from microcrystalline cellulose, mannitol, lactose, pregelatinized starch, sodium starch glycolate, corn starch or derivatives thereof, povidone, crospovidone, calcium stearate, glyceryl monostearate, glyceryl palmitostearate, talc, colloidal silicone dioxide, magnesium stearate, sodium lauryl sulfate, sodium stearyl fumarate, zinc stearate, stearic acid or hydrogenated vegetable oil, gum arabica, magnesia, glucose, fats, waxes, natural or hardened oils, water, physiological sodium chloride solution or alcohols, for example, ethanol, propanol or glycerol, sugar solutions, such as glucose solutions or mannitol solutions and the like or a mixture of the various excipients.

In another embodiment, the pharmaceutical compositions of the invention may be formulated in the form of pills, tablets, coated tablets, capsules, powder, granules, pellets, patches, implants, films, liquids, semi-solids, gels, aerosols, emulsions, elixirs and the like. Such pharmaceutical compositions and processes for preparing same are well known in the art.

In another embodiment, the pharmaceutical composition of the instant invention contains 1 to 90 %, 5 to 75 %, and 10 to 60 % by weight of the masupirdine of the instant invention or a pharmaceutically acceptable salt thereof. The amount of the masupirdine or its pharmaceutically acceptable salt in the pharmaceutical composition(s) can range from about 0.1 mg to about 100 mg or from about 0.1 mg to about 60 mg or from about 0.1 mg to about 30 mg or in any range falling within the broader range of 0.1 mg to 100 mg.

In another embodiment, the pharmaceutical composition of the instant invention can be conventional formulations such as immediate-release formulations, modified release formulations such as sustained-release formulations, delayedrelease formulations, and extended-release formulations, or new delivery systems such as oral disintegrating formulations and transdermal patches.

The dose of the compounds can vary depending on factors such as age and weight of patient, nature, route of administration, and severity of the disease to be treated and such other factors.

In embodiments, the pharmaceutical composition of the present invention can be administered to the patient once a day, twice a day, thrice a day, preferably once a day or twice a day, more preferably once a day.

In embodiments, the pharmaceutical composition of the present invention can be administered to the patient by oral, nasal, local, or parenteral routes.

### EXAMPLES

### Abbreviations:

- AD :: Alzheimer's disease
- ADAS-Cog :: Alzheimer's Disease Assessment Scale-Cognitive Subscale
- bid :: Bis in die (twice daily)
- C-SDD :: Cornell Scale for Depression in Dementia
- HCl :: Hydrochloric acid
- mITT :: Modified Intention-To-Treat
- MDD :: Major depressive disorder
- MMSE :: Mini-Mental State Examination
- MMRM :: Mixed Model Repeated Measures
- mg :: Milligram
- mL :: Milliliter
- NPI :: Neuropsychiatric Inventory
- NINCDS-ADRDA :: National Institute of Neurological and Communicative Diseases and Stroke/Alzheimer's Disease and Related Disorders Association
- ng :: Nanogram
- qd :: Quaque die (Once daily)
- ≥ :: Greater than or equal to
- Δ :: Difference

The following examples are offered for illustrative purposes, and are not intended to limit the invention in any manner.

### Example 1:

This example describes a phase 2A, proof-of-concept, 26-week, double-blind, multicenter, randomized, parallel group, placebo-controlled study to compare the efficacy and safety of treatment with masupirdine (50 mg or 100 mg once daily [qd]) to placebo treatment in subjects with moderate AD (MMSE score of 12 to 20, both inclusive). The study consisted of a 2 to 4-week screening period, followed by a 26-week double-blind treatment period and a 4-week single-blind placebo washout period.

**Number of subjects:** A total of 564 subjects were enrolled and randomized into one of three treatment groups: masupirdine 50 mg, masupirdine 100 mg, or placebo.

**Population:** The study population included male or female subjects, 50 to 85 years of age, with moderate dementia due to probable AD (MMSE 12-20, both inclusive) based on the National Institute of Neurological and Communicative Diseases and Stroke/Alzheimer's Disease and Related Disorders Association (NINCDS-ADRDA) criteria, diagnosed at least 1 year prior to the study and receiving stable doses for at least 3 months of donepezil HCl (10 mg qd) and either memantine HCl (10 mg bid) or Namenda XR^{®} (28 mg memantine HCl extended-release qd) or the combination therapy, Namzaric^{™} (28 mg memantine HCl extended-release / 10 mg donepezil HCl) qd.

**Treatment:** Eligible subjects received double-blind oral administration of one of three treatments: masupirdine (50 mg qd), masupirdine (100 mg qd), or placebo (qd) in a 1:1:1 ratio. Throughout the study, all subjects continued to receive donepezil HCl (10 mg qd) and memantine HCl (10 mg bid) or Namenda XR^{®} (28 mg memantine HCl extended-release qd) or the combination therapy, Namzaric^{™} (28 mg memantine HCl extended-release / 10 mg donepezil HCl) qd.

Subjects were treated with masupirdine or placebo for a total of 26 weeks followed by a 4-week single-blind placebo washout period. During the washout period, all subjects were received placebo in addition to donepezil HCl and memantine HCl.

Masupirdine was supplied as identically appearing tablets that contain either 50 mg or 100 mg of masupirdine. Placebo tablets matching masupirdine tablets were also supplied.

Subjects and caregivers must meet all the inclusion criteria and none of the exclusion criteria.

**Inclusion Criteria:** Subjects must meet all of the following inclusion criteria to be enrolled into the study:
1. Male or female subjects, aged between 50 and 85 years inclusive at screening.
2. Has a diagnosis of probable Alzheimer's disease based on the NINCDS-ADRDA criteria at least 1 year prior to the screening visit.
3. Has a score between 12 and 20 inclusive on the MMSE at the screening and baseline visits.
4. Must be receiving treatment with stable doses of donepezil HCl (10 mg qd), either as 10 mg donepezil HCl only or part of the combination therapy, Namzaric^{™} (28 mg memantine HCl extended-release / 10 mg donepezil HCl) qd for at least 3 months prior to screening visit. Subjects are likely to be maintained on this 10 mg daily dose of donepezil HCl or Namzaric^{™} for the entire duration of the study.
5. Must be receiving treatment with stable doses of memantine HCl (10 mg bid) or Namenda XR^{®} (28 mg memantine HCl extended-release qd) or as part of the combination therapy, Namzaric^{™} (28 mg memantine HCl extended-release / 10 mg donepezil HCl) qd for at least 3 months prior to the screening visit. Subjects are likely to be maintained on their current dose of memantine HCl or Namenda XR^{®} or Namzaric^{™} for the duration of the study.
6. Availability of a person (caregiver) who in the investigator's judgment has frequent and sufficient contact with the subject, such that this person is qualified, willing and able to provide accurate information regarding the subject's cognitive and functional abilities and will accompany the subject to study visits. The caregiver should have face to face contact with the subject for a minimum of approximately 12 hours per week spread over 3 to 5 days during the week (for example: 3 hours per day for 4 days a week, or 4 hours per day for 3 days a week).

**Exclusion Criteria** Subjects who meet any of the following exclusion criteria were not allowed to be randomized into the study:
1. Has a diagnosis of schizophrenia, bipolar disorder or current major depressive disorder (MDD) or subjects whose C-SDD scores are suggestive of probable depression (typically scores ≥12). Subjects with history of MDD who are currently treated and controlled on medication for at least 6 months may be enrolled. Subjects taking low doses of antipsychotics for the treatment of sleep disturbances or for agitation or aggression, for which the dose has been stable for at least 1 month and not anticipated to change during the course of the study, can be enrolled.
2. Is treated or likely to require treatment during the study, with any medications prohibited by the study protocol.

### Criteria for evaluation of efficacy and safety:

The primary efficacy variable was ADAS-Cog 11 and a key secondary efficacy variable was NPI-12.

### ADAS-Cog 11 assessments procedure:

The Alzheimer's Disease Assessment Scale-Cognitive Subscale (ADAS-Cog) was developed to assess the level of cognitive dysfunction in AD. It is also used for assessing the efficacy of anti-dementia treatments. The ADAScog-11 is a brief measure of cognition, including word recall, commands, constructional praxis, naming objects and fingers, ideational praxis, orientation, word recognition task, remembering test instructions, spoken language ability, word-finding difficulty, and comprehension of spoken language. The test is scored by an administrator who adds up points for the errors in each task for a total score ranging from 0 to 70. The greater the dysfunction, the greater the score. A score of 70 represents the most severe impairment.

### NPI assessments procedure:

The NPI is a 12-item structured interview with a caregiver designed to assess behavioral disturbances in patients with dementia. The NPI comprises 10 behavioral (hallucinations, delusions, agitation/aggression, dysphoria/depression, anxiety, irritability, disinhibition, euphoria/elation, apathy and aberrant motor behavior), and neurovegetative items (sleep and night-time behavior change, appetite and eating change). The behavioral domains are assessed in terms of frequency, severity and distress. Each item consists of a screening question and several sub-questions which are rated no (not present) or yes (present). Each item is then rated for frequency (a 4-point scale from 1 [occasionally] to 4 [very frequently]), severity (a 3-point scale from 1 [mild] to 3 [marked]), and caregiver distress (a 6-point scale from 0 [not at all] to 5 [very severely or extremely]). The total score12 items of the frequency and severity ratings range from 0 to 144 and the total score12 items of caregiver distress ranges from 0 to 60. The NPI was administered to the subject's caregiver and scored by a qualified, trained rater at baseline and Weeks 4, 13 and 26.

The safety assessed by monitoring adverse events, physical examinations, vital signs, clinical laboratory tests and electrocardiograms.

### Statistical Analysis:

Data was analyzed using a mixed effects model for repeated measures (MMRM). The model included fixed categorical factors for treatment, week, treatment-by-week interaction, and APO-E status (carrier- one allele, carrier- two alleles, and noncarrier), as well as a continuous covariate of baseline score, the baseline score-by-week interaction, age, and baseline MMSE score. In subgroup analysis, stratification was based on the baseline symptoms and/or symptom emergence.

### Results:

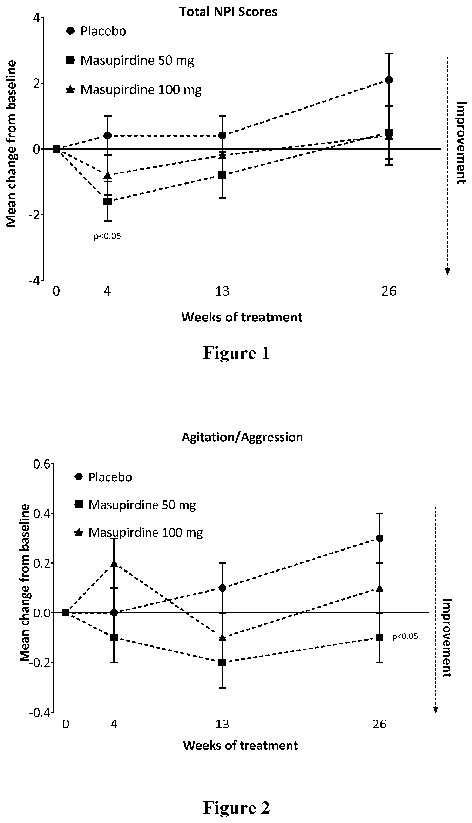

The results of the study are discussed below:
Masupirdine was safe and well tolerated with no significant adverse events and the primary efficacy endpoint (26 week change from baseline as measured by ADAS-Cog 11) was not achieved. However, the mean NPI-12 scores slightly decreased in the masupirdine, 50 mg arm, showing a statistically significant treatment differences in change from baseline, when comparing masupirdine, 50 mg arm to the placebo arm at week 4. At Week 13, the mean NPI-12 scores remained similar to baseline across all 3 treatment arms. At Week 26, the mean NPI-12 scores slightly increased in the placebo arm, however, the scores remained similar to baseline in the masupirdine dose groups (Figure 1).

At Week 26, a statistically significant treatment difference in change from baseline in the mean agitation/aggression domain scores of NPI-12 was observed, when comparing masupirdine 50 mg arm to the placebo arm (Figure 2).

Based on the significant treatment effects on NPI-12 total scores and agitation/aggression domain scores, exploratory subgroup analyses of the twelve domains of NPI were carried out to understand the beneficial effects of masupirdine on the neuropsychiatric symptoms.

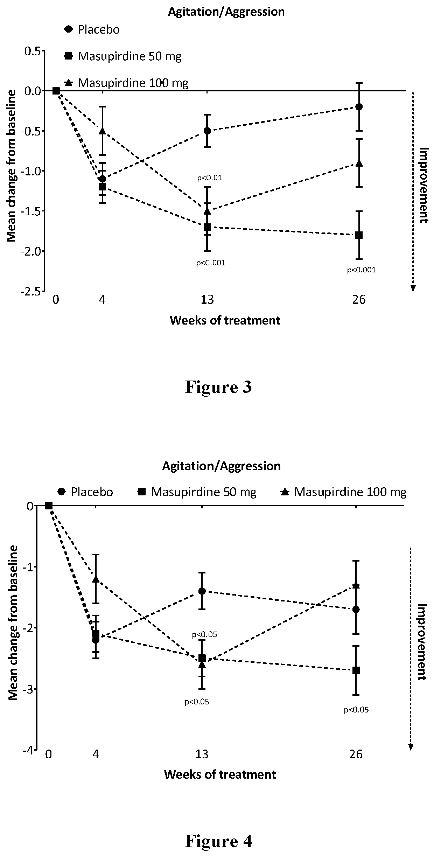

Subgroup analysis of NPI-12 domain agitation/aggression by baseline domain score ≥ 1 (baseline symptoms) in the mITT population showed a statistically significant treatment difference in change from baseline in the mean agitation/aggression scores at Week 13 when comparing each masupirdine dose arm to the placebo arm. A statistically significant treatment difference was also observed at Week 26, when comparing the masupirdine, 50 mg arm to the placebo arm. Similar results were seen when treatment was added as a covariate. When treatment and time were added as covariates in the mITT population, statistically significant treatment differences were observed (Figure 3).

Subgroup analysis of NPI-12 domain agitation/aggression by baseline domain score ≥ 3 (baseline symptoms) in the mITT population showed a statistically significant treatment difference in change from baseline in the mean agitation/aggression scores at Week 13 and 26, when comparing masupirdine, 50 mg arm to the placebo arm. At Week 13, a statistically significant treatment difference was also noted in the masupirdine, 100 mg arm compared to placebo. When treatment was added as covariate, a statistically significant treatment difference in change from baseline at Week 26 was observed, when comparing the masupirdine, 50 mg arm to the placebo arm. When treatment and time were added as covariates, statistically significant treatment differences were observed (Figure 4).

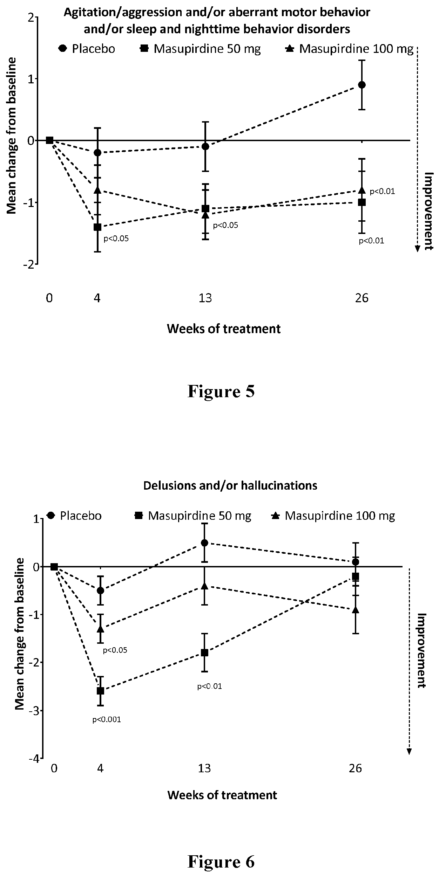

Subgroup analysis of NPI-12 domain extended composite NPI-12 score domain (agitation/aggression, aberrant motor behavior and sleep and nighttime behavior disorders) by baseline domain score ≥ 1 (baseline symptoms) in the mITT population showed statistically significant treatment differences when comparing masupirdine, 50 mg arm to the placebo arm at Week 4, when comparing the masupirdine, 100 mg arm to the placebo arm at Week 13, and when comparing each masupirdine dose arm to the placebo arm at Week 26. When treatment was added as covariate, a statistically significant treatment difference was observed, when comparing the masupirdine, 50 mg arm to the placebo arm (Figure 5).

Subgroup analysis of NPI-12 domain delusions and/or hallucinations by baseline domain score ≥ 1 (baseline symptoms) in the mITT population showed a statistically significant treatment difference in change from baseline at Week 4 and Week 13, when comparing each masupirdine dose arm to the placebo arm, and masupirdine 50 mg arm to the placebo arm, respectively. When treatment was added as covariate, a statistically significant treatment difference was observed, when comparing masupirdine 100 mg arm to the placebo arm. When treatment and time were added as covariates, statistically significant treatment differences were observed (Figure 6).

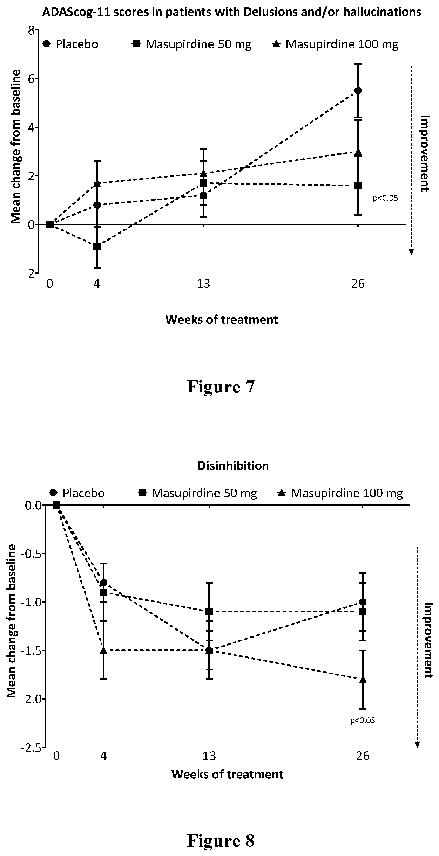

Subgroup analysis of ADAScog-11 scores by baseline delusion and/or hallucination scores in subjects with baseline delusion and/or hallucination score ≥1 showed a statistically significant treatment difference in change from baseline in ADAScog-11 scores at Week 26, when comparing masupirdine 50 mg arm to the placebo arm in the mITT population. When treatment was added as covariate, a statistically significant treatment difference was observed, when comparing masupirdine 50 mg arm to the placebo arm. When treatment and time were added as covariates, statistically significant treatment differences were observed, when comparing masupirdine 50 mg dose arm to the placebo arm (Figure 7).

Subgroup analysis of NPI-12 domain disinhibition by baseline domain score ≥ 1 (baseline symptoms) in the mITT population showed a statistically significant treatment difference in change from baseline at Week 26, when comparing masupirdine 100 mg arm to the placebo arm (Figure 8).

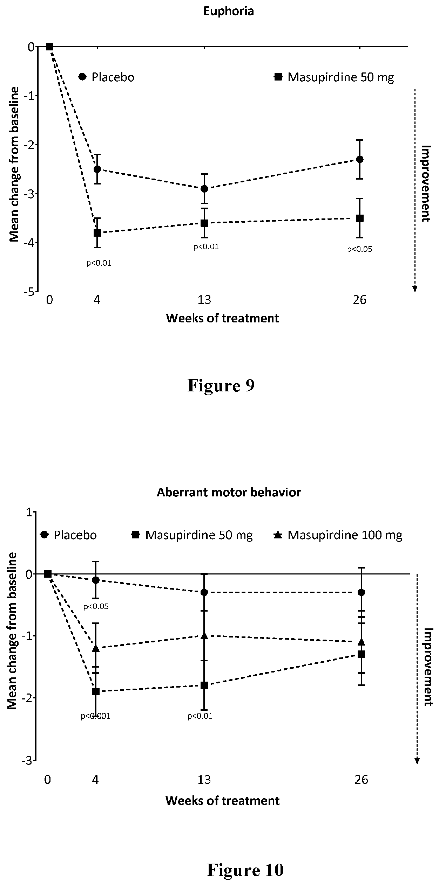

Subgroup analysis of NPI-12 domain euphoria/elation by baseline domain score ≥ 1 (baseline symptoms) in the mITT population showed a statistically significant treatment differences in change from baseline in the mean euphoria/elation scores at Week 4, Week 13 and Week 26 when comparing masupirdine 50 mg dose arm to the placebo arm. Similar results were seen when treatment and time were added as covariates (Figure 9).

Subgroup analysis of NPI-12 domain aberrant motor behavior by baseline domain score ≥ 1 (baseline symptoms) in the mITT population showed a statistically significant treatment differences in change from baseline at Week 4, when comparing masupirdine 50 mg dose arm to the placebo arm. At Week 13, a statistically significant treatment difference was observed when comparing masupirdine 50 mg arm to the placebo (Figure 10).

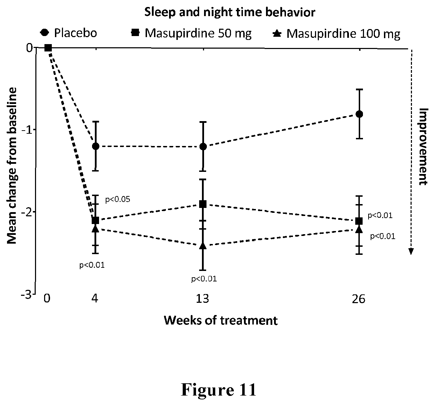

Subgroup analysis of NPI-12 domain sleep and nighttime behavior by baseline domain score ≥ 1 (baseline symptoms) showed a statistically significant treatment difference in change from baseline at Week 4, when comparing each masupirdine dose arm to the placebo arm in the mITT population. At Week 13, a statistically significant treatment difference in change from baseline was observed, when comparing masupirdine 100 mg arm to the placebo arm. At Week 26, statistically significant treatment differences in change from baseline was observed when comparing each masupirdine dose arm to the placebo arm. When treatment was added as covariate, a statistically significant treatment difference in change from baseline at Week 26 was observed, when comparing each masupirdine dose arm to the placebo arm in both the populations (Figure 11).

In summary, beneficial effects of masupirdine was observed in various domains of neuropsychiatric symptoms when assessed using the NPI-12 scale.

## Claims

1. Masupirdine, or a pharmaceutically acceptable salt thereof, for use in a method of treating agitation/aggression, delusions, hallucinations, delusions and hallucinations, aberrant motor behavior, euphoria/elation, disinhibition, or sleep and night-time behavior change in a patient with dementia, comprising administering to the patient an effective dose of masupirdine or a pharmaceutically acceptable salt thereof, wherein the effective dose of masupirdine or a pharmaceutically acceptable salt thereof is selected from 25 mg to 125 mg, wherein the dementia is selected from Alzheimer's disease dementia, dementia associated with schizophrenia, Parkinson's disease dementia, Lewy body dementia, vascular dementia, or frontotemporal dementia.

2. Masupirdine, or a pharmaceutically acceptable salt thereof, for the use of claim 1, wherein the treatment comprises administering to the patient 25 mg to 50 mg of masupirdine or a pharmaceutically acceptable salt thereof; or
wherein the treatment comprises administering to the patient 50 mg to 100 mg of masupirdine or a pharmaceutically acceptable salt thereof; or
wherein the treatment comprises administering to the patient 25 mg, 50 mg, 75 mg, 100 mg or 125 mg of masupirdine or a pharmaceutically acceptable salt thereof.

3. Masupirdine, or a pharmaceutically acceptable salt thereof, for the use of claim 1 or claim 2, wherein the pharmaceutically acceptable salt of masupirdine is dimesylate monohydrate.

4. Masupirdine, or a pharmaceutically acceptable salt thereof, for the use of claim 1, comprising administering to the patient an effective dose of masupirdine dimesylate monohydrate, wherein the effective dose of masupirdine dimesylate monohydrate is selected from 25 mg to 125 mg.

5. Masupirdine, or a pharmaceutically acceptable salt thereof, for the use of claim 4, wherein the treatment comprises administering to the patient 25 mg, 50 mg, 75 mg, 100 mg or 125 mg of masupirdine dimesylate monohydrate.

6. Masupirdine, or a pharmaceutically acceptable salt thereof, for the use of claim 1 or claim 2, wherein the treatment comprises administering to the patient an effective dose of masupirdine or a pharmaceutically acceptable salt thereof in combination with donepezil or a pharmaceutically acceptable salt thereof and memantine or a pharmaceutically acceptable salt thereof.

7. Masupirdine, or a pharmaceutically acceptable salt thereof, for the use of any one of claims 1 to 3, for the treatment of agitation/aggression in a patient with Alzheimer's disease dementia.

8. Masupirdine, or a pharmaceutically acceptable salt thereof, for the use of any one of claims 1 to 3, for the treatment of delusions, hallucinations, or delusions and hallucinations in a patient with Alzheimer's disease dementia.

9. A pharmaceutical composition, comprising an effective dose of masupirdine or a pharmaceutically acceptable salt thereof, for use in the treatment of agitation/aggression, delusions, hallucinations, delusions and hallucinations, aberrant motor behavior, euphoria/elation, disinhibition, or sleep and nighttime behavior change, in a patient with dementia, wherein the effective dose of masupirdine or a pharmaceutically acceptable salt thereof is selected from 25 mg to 125 mg, wherein the dementia is selected from Alzheimer's disease dementia, dementia associated with schizophrenia, Parkinson's disease dementia, Lewy body dementia, vascular dementia, or frontotemporal dementia.

10. The pharmaceutical composition, comprising an effective dose of masupirdine or a pharmaceutically acceptable salt thereof, for use of claim 9, wherein the effective dose of masupirdine or a pharmaceutically acceptable salt thereof is selected from 25 mg to 50 mg; or
wherein the effective dose of masupirdine or a pharmaceutically acceptable salt thereof is selected from 50 mg to 100 mg; or
wherein the effective dose of masupirdine or a pharmaceutically acceptable salt thereof is selected from 25 mg, 50 mg, 75 mg, 100 mg or 125 mg.

11. The pharmaceutical composition, comprising an effective dose of masupirdine or a pharmaceutically acceptable salt thereof, for use as claimed in claim 9 or claim 10, wherein the pharmaceutically acceptable salt of masupirdine is dimesylate monohydrate.

12. The pharmaceutical composition for use of claim 9, for use in combination with donepezil or a pharmaceutically acceptable salt thereof and memantine or a pharmaceutically acceptable salt thereof.

13. The pharmaceutical composition for use of any one of claims 9 to 12, wherein the agitation/aggression is in a patient with Alzheimer's disease dementia.

14. The pharmaceutical composition for use of any one of claims 9 to 12, wherein the delusions, hallucinations, or delusions and hallucinations are in a patient with Alzheimer's disease dementia.

15. Masupirdine, or a pharmaceutically acceptable salt thereof, for the use of claim 6, or the pharmaceutical composition for use of any one of claims 9 to 12, wherein the pharmaceutically acceptable salt of masupirdine is dimesylate monohydrate.

16. Masupirdine, or a pharmaceutically acceptable salt thereof, for the use of claim 6, or the pharmaceutical composition for use of claim 12, wherein the pharmaceutically acceptable salt of donepezil is hydrochloride.

17. Masupirdine, or a pharmaceutically acceptable salt thereof, for the use of claim 6, or the pharmaceutical composition for use of claim 12, wherein the pharmaceutically acceptable salt of memantine is hydrochloride.

## Patentansprüche

1. Masupirdin oder pharmazeutisch unbedenkliches Salz davon zur Verwendung in einem Verfahren zur Behandlung von Agitation/Aggression, Wahnvorstellungen, Halluzinationen, Wahnvorstellungen und Halluzinationen, ziellosem motorischem Verhalten, Euphorie/Hochgefühl, Enthemmung oder einer Änderung des Schlaf- und Nachtverhaltens bei einem Patienten mit Demenz, das Verabreichen einer wirksamen Dosis von Masupirdin oder eines pharmazeutisch unbedenklichen Salzes davon an den Patienten umfasst, wobei die wirksame Dosis von Masupirdin oder eines pharmazeutisch unbedenklichen Salzes davon aus 25 mg bis 125 mg ausgewählt ist, wobei die Demenz aus Demenz bei Alzheimer-Krankheit, mit Schizophrenie assoziierter Demenz, Demenz bei Parkinson-Krankheit, Lewy-Körper-Demenz, vaskulärer Demenz oder frontotemporaler Demenz ausgewählt ist.

2. Masupirdin oder pharmazeutisch unbedenkliches Salz davon zur Verwendung nach Anspruch 1, wobei die Behandlung Verabreichen von 25 mg bis 50 mg Masupirdin oder eines pharmazeutisch unbedenklichen Salzes davon an den Patienten umfasst; oder
wobei die Behandlung Verabreichen von 50 mg bis 100 mg Masupirdin oder eines pharmazeutisch unbedenklichen Salzes davon an den Patienten umfasst; oder
wobei die Behandlung Verabreichen von 25 mg, 50 mg, 75 mg, 100 mg oder 125 mg Masupirdin oder eines pharmazeutisch unbedenklichen Salzes davon an den Patienten umfasst.

3. Masupirdin oder pharmazeutisch unbedenkliches Salz davon zur Verwendung nach Anspruch 1 oder Anspruch 2, wobei das pharmazeutisch unbedenkliche Salz von Masupirdin Dimesylat-Monohydrat ist.

4. Masupirdin oder pharmazeutisch unbedenkliches Salz davon zur Verwendung nach Anspruch 1, umfassend Verabreichen einer wirksamen Dosis von Masupirdin-Dimesylat-Monohydrat an den Patienten, wobei die wirksame Dosis von Masupirdin-Dimesylat-Monohydrat aus 25 mg bis 125 mg ausgewählt ist.

5. Masupirdin oder pharmazeutisch unbedenkliches Salz davon zur Verwendung nach Anspruch 4, wobei die Behandlung Verabreichen von 25 mg, 50 mg, 75 mg, 100 mg oder 125 mg Masupirdin-Dimesylat-Monohydrat an den Patienten umfasst.

6. Masupirdin oder pharmazeutisch unbedenkliches Salz davon zur Verwendung nach Anspruch 1 oder Anspruch 2, wobei die Behandlung Verabreichen einer wirksamen Dosis von Masupirdin oder eines pharmazeutisch unbedenklichen Salzes davon in Kombination mit Donepezil oder einem pharmazeutisch unbedenklichen Salz davon und Memantin oder einem pharmazeutisch unbedenklichen Salz davon an den Patienten umfasst.

7. Masupirdin oder pharmazeutisch unbedenkliches Salz davon zur Verwendung nach einem der Ansprüche 1 bis 3 zur Behandlung von Agitation/Aggression bei einem Patienten mit Demenz bei Alzheimer-Krankheit.

8. Masupirdin oder pharmazeutisch unbedenkliches Salz davon zur Verwendung nach einem der Ansprüche 1 bis 3 zur Behandlung von Wahnvorstellungen, Halluzinationen oder Wahnvorstellungen und Halluzinationen bei einem Patienten mit Demenz bei Alzheimer-Krankheit.

9. Pharmazeutische Zusammensetzung, umfassend eine wirksame Dosis von Masupirdin oder eines pharmazeutisch unbedenklichen Salzes davon, zur Verwendung bei der Behandlung von Agitation/Aggression, Wahnvorstellungen, Halluzinationen, Wahnvorstellungen und Halluzinationen, ziellosem motorischem Verhalten, Euphorie/Hochgefühl, Enthemmung oder einer Änderung des Schlaf- und Nachtverhaltens bei einem Patienten mit Demenz, wobei die wirksame Dosis von Masupirdin oder eines pharmazeutisch unbedenklichen Salzes davon aus 25 mg bis 125 mg ausgewählt ist, wobei die Demenz aus Demenz bei Alzheimer-Krankheit, mit Schizophrenie assoziierter Demenz, Demenz bei Parkinson-Krankheit, Lewy-Körper-Demenz, vaskulärer Demenz oder frontotemporaler Demenz ausgewählt ist.

10. Pharmazeutische Zusammensetzung, umfassend eine wirksame Dosis von Masupirdin oder eines pharmazeutisch unbedenklichen Salzes davon, zur Verwendung nach Anspruch 9, wobei die wirksame Dosis von Masupirdin oder eines pharmazeutisch unbedenklichen Salzes davon aus 25 mg bis 50 mg ausgewählt ist; oder
wobei die wirksame Dosis von Masupirdin oder eines pharmazeutisch unbedenklichen Salzes davon aus 50 mg bis 100 mg ausgewählt ist; oder
wobei die wirksame Dosis von Masupirdin oder eines pharmazeutisch unbedenklichen Salzes davon aus 25 mg, 50 mg, 75 mg, 100 mg oder 125 mg ausgewählt ist.

11. Pharmazeutische Zusammensetzung, umfassend eine wirksame Dosis von Masupirdin oder eines pharmazeutisch unbedenklichen Salzes davon, zur Verwendung nach Anspruch 9 oder Anspruch 10, wobei das pharmazeutisch unbedenkliche Salz von Masupirdin Dimesylat-Monohydrat ist.

12. Pharmazeutische Zusammensetzung zur Verwendung nach Anspruch 9 zur Verwendung in Kombination mit Donepezil oder einem pharmazeutisch unbedenklichen Salz davon und Memantin oder einem pharmazeutisch unbedenklichen Salz davon.

13. Pharmazeutische Zusammensetzung zur Verwendung nach einem der Ansprüche 9 bis 12, wobei die Agitation/Aggression bei einem Patienten mit Demenz bei Alzheimer-Krankheit auftritt.

14. Pharmazeutische Zusammensetzung zur Verwendung nach einem der Ansprüche 9 bis 12, wobei die Wahnvorstellungen, Halluzinationen oder Wahnvorstellungen und Halluzinationen bei einem Patienten mit Demenz bei Alzheimer-Krankheit auftreten.

15. Masupirdin oder pharmazeutisch unbedenkliches Salz davon zur Verwendung nach Anspruch 6 oder pharmazeutische Zusammensetzung zur Verwendung nach einem der Ansprüche 9 bis 12, wobei das pharmazeutisch unbedenkliche Salz von Masupirdin Dimesylat-Monohydrat ist.

16. Masupirdin oder pharmazeutisch unbedenkliches Salz davon zur Verwendung nach Anspruch 6 oder pharmazeutische Zusammensetzung zur Verwendung nach Anspruch 12, wobei das pharmazeutisch unbedenkliche Salz von Donepezil Hydrochlorid ist.

17. Masupirdin oder pharmazeutisch unbedenkliches Salz davon zur Verwendung nach Anspruch 6 oder pharmazeutische Zusammensetzung zur Verwendung nach Anspruch 12, wobei das pharmazeutisch unbedenkliche Salz von Memantin Hydrochlorid ist.

## Revendications

1. Masupirdine, ou sel acceptable pharmaceutiquement de celle-ci, destiné(e) à être utilisé(e) dans un procédé de traitement de l'agitation/agression, des délires, des hallucinations, des délires et hallucinations, du comportement moteur aberrant, de l'euphorie/exaltation, de la désinhibition ou du changement du sommeil et du comportement nocturne chez un patient atteint de démence, comprenant l'administration au patient d'une dose efficace de masupirdine ou d'un sel acceptable pharmaceutiquement de celle-ci, ladite dose efficace de masupirdine ou d'un sel acceptable pharmaceutiquement de celle-ci étant choisie de 25 mg à 125 mg, ladite démence étant choisie parmi la démence de la maladie d'Alzheimer, la démence associée à la schizophrénie, la démence de la maladie de Parkinson, la démence à corps de Lewy, la démence vasculaire ou la démence frontotemporale.

2. Masupirdine, ou sel acceptable pharmaceutiquement de celle-ci, destiné(e) à être utilisé(e) selon la revendication 1, ledit traitement comprenant l'administration au patient de 25 mg à 50 mg de masupirdine ou d'un sel acceptable pharmaceutiquement de celle-ci ; ou
ledit traitement comprenant l'administration au patient de 50 mg à 100 mg de masupirdine ou d'un sel acceptable pharmaceutiquement de celle-ci ; ou
ledit traitement comprenant l'administration au patient de 25 mg, 50 mg, 75 mg, 100 mg ou 125 mg de masupirdine ou d'un sel acceptable pharmaceutiquement de celle-ci.

3. Masupirdine, ou sel acceptable pharmaceutiquement de celle-ci, destiné(e) à être utilisé(e) selon la revendication 1 ou la revendication 2, ledit sel acceptable pharmaceutiquement de masupirdine étant le dimésylate monohydraté.

4. Masupirdine, ou sel acceptable pharmaceutiquement de celle-ci, destiné(e) à être utilisé(e) selon la revendication 1, comprenant l'administration au patient d'une dose efficace de dimésylate monohydraté de masupirdine, ladite dose efficace de dimésylate monohydraté de masupirdine étant choisie de 25 mg à 125 mg.

5. Masupirdine, ou sel acceptable pharmaceutiquement de celle-ci, destiné(e) à être utilisé(e) selon la revendication 4, ledit traitement comprenant l'administration au patient de 25 mg, 50 mg, 75 mg, 100 mg ou 125 mg de dimésylate monohydraté de masupirdine.

6. Masupirdine, ou sel acceptable pharmaceutiquement de celle-ci, destiné(e) à être utilisé(e) selon la revendication 1 ou la revendication 2, ledit traitement comprenant l'administration au patient d'une dose efficace de masupirdine ou d'un sel acceptable pharmaceutiquement de celle-ci en combinaison avec le donépézil ou un sel acceptable pharmaceutiquement de celui-ci et la mémantine ou un sel acceptable pharmaceutiquement de celle-ci.

7. Masupirdine, ou sel acceptable pharmaceutiquement de celle-ci, destiné(e) à être utilisé(e) selon l'une quelconque des revendications 1 à 3, pour le traitement de l'agitation/agression chez un patient atteint de démence de la maladie d'Alzheimer.

8. Masupirdine, ou sel acceptable pharmaceutiquement de celle-ci, destiné(e) à être utilisé(e) selon l'une quelconque des revendications 1 à 3, pour le traitement des délires, des hallucinations, ou des délires et hallucinations chez un patient atteint de démence de la maladie d'Alzheimer.

9. Composition pharmaceutique, comprenant une dose efficace de masupirdine ou d'un sel acceptable pharmaceutiquement de celle-ci, destinée à être utilisée dans le traitement de l'agitation/agression, des délires, des hallucinations, des délires et hallucinations, du comportement moteur aberrant, de l'euphorie/exaltation, de la désinhibition ou du changement du sommeil et du comportement nocturne chez un patient atteint de démence, ladite dose efficace de masupirdine ou d'un sel acceptable pharmaceutiquement de celle-ci est choisie de 25 mg à 125 mg, ladite démence est choisie parmi la démence de la maladie d'Alzheimer, la démence associée à la schizophrénie, la démence de la maladie de Parkinson, la démence à corps de Lewy, la démence vasculaire ou la démence frontotemporale.

10. Composition pharmaceutique, comprenant une dose efficace de masupirdine ou d'un sel acceptable pharmaceutiquement de celle-ci, destinée à être utilisée selon la revendication 9, dans laquelle la dose efficace de masupirdine ou d'un sel acceptable pharmaceutiquement de celle-ci est choisie de 25 mg à 50 mg ; ou
dans laquelle la dose efficace de masupirdine ou d'un sel acceptable pharmaceutiquement de celle-ci est choisie de 50 mg à 100 mg ; ou
dans laquelle la dose efficace de masupirdine ou d'un sel acceptable pharmaceutiquement de celle-ci est choisie parmi 25 mg, 50 mg, 75 mg, 100 mg ou 125 mg.

11. Composition pharmaceutique, comprenant une dose efficace de masupirdine ou d'un sel acceptable pharmaceutiquement de celle-ci, destinée à être utilisée selon la revendication 9 ou la revendication 10, dans laquelle le sel acceptable pharmaceutiquement de masupirdine est le dimésylate monohydraté.

12. Composition pharmaceutique destinée à être utilisée selon la revendication 9, destinée à être utilisée en combinaison avec le donépézil ou un sel acceptable pharmaceutiquement de celui-ci et la mémantine ou un sel acceptable pharmaceutiquement de celle-ci.

13. Composition pharmaceutique destinée à être utilisée selon l'une quelconque des revendications 9 à 12, dans laquelle l'agitation/agression concerne un patient atteint de démence de la maladie d'Alzheimer.

14. Composition pharmaceutique destinée à être utilisée selon l'une quelconque des revendications 9 à 12, dans laquelle les délires, les hallucinations ou les délires et hallucinations concernent un patient atteint de démence de la maladie d'Alzheimer.

15. Masupirdine, ou sel acceptable pharmaceutiquement de celle-ci, destiné(e) à être utilisé(e) selon la revendication 6, ou composition pharmaceutique destinée à être utilisée selon l'une quelconque des revendications 9 à 12, ledit sel acceptable pharmaceutiquement de masupirdine étant le dimésylate monohydraté.

16. Masupirdine, ou sel acceptable pharmaceutiquement de celle-ci, destiné(e) à être utilisé(e) selon la revendication 6, ou composition pharmaceutique destinée à être utilisée selon la revendication 12, ledit sel acceptable pharmaceutiquement de donépézil étant le chlorhydrate.

17. Masupirdine, ou sel acceptable pharmaceutiquement de celle-ci, destiné(e) à être utilisé(e) selon la revendication 6, ou composition pharmaceutique destinée à être utilisée selon la revendication 12, ledit sel acceptable pharmaceutiquement de mémantine étant le chlorhydrate.
